# EUROPEAN PATENT APPLICATION

(11) **EP 3 456 876 A1**
(43) Date of publication of application: **20.03.2019**
(21) Application number: 16829223.3
(22) Date of filing: 11.05.2016
(51) Int. Cl.: D07B 1/06

(54) **WIRE ROPE**

(71) Applicant: Asahi Intecc Co., Ltd., Seto-shi, Aichi 489-0071 (JP)
(72) Inventor: HASHIMOTO, Takaya, Nagoya-shi Aichi 463-0024 (JP)
(74) Representative: Winter, Brandl, Fürniss, Hübner, Röss, Kaiser, Polte - Partnerschaft mbB
(86) International application number: PCT/JP2016/063945
(87) International publication number: WO 2017/195284

(57) **Abstract**

[Problem] An object is to provide a wire rope having an improved durability without use of grease thereinside, in particular, a wire rope having an improved durability which can be used in a medical device to be inserted into a patient's body.

[Solution] A wire rope 1 comprises a core wire 3 and 6 side wires 5, in which the core wire 3 (a special metal element wire) has a hardness of the outer periphery in a cross-section higher than that of the center in the cross-section.

## Description

### TECHNICAL FILED

The present invention relates to a wire rope comprising multiple metal element wires.

### BACKGROUND ART

To date, wire ropes have been known in each of which multiple metal element wires are twisted. In general, wire ropes have advantages such as excellent impact resistance and good flexibility as compared with a single metal element wire. Further, wire ropes are often subjected to repeated bending, and thus desirably have good durability under such usage conditions.

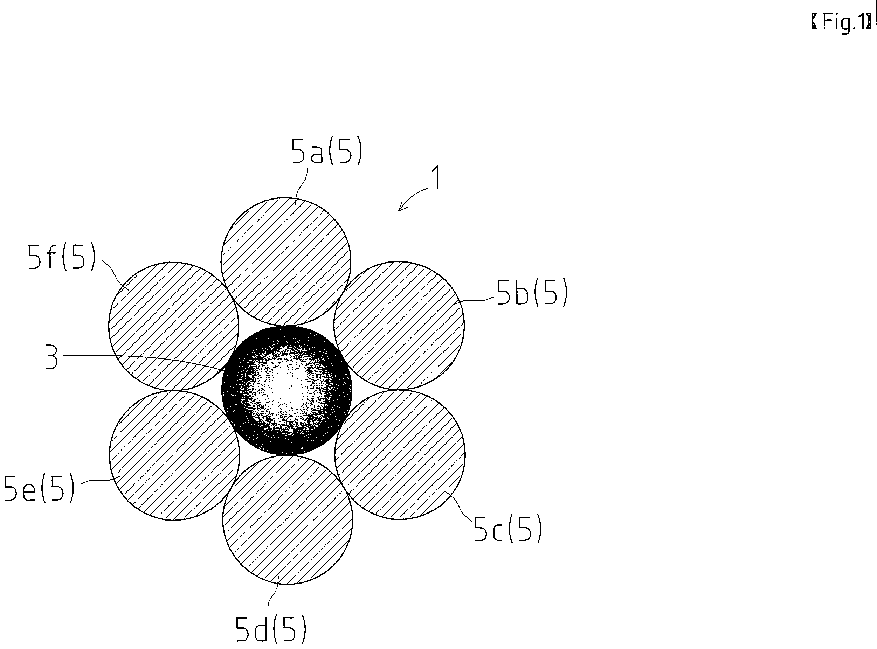

For example, Patent Document 1 describes an aluminum twisted wire (a wire rope) in which spaces between twisted wires inside the outermost aluminum twisted wire layer 14 is filled with grease 6 to improve its life time (see Fig. 1 and the like).

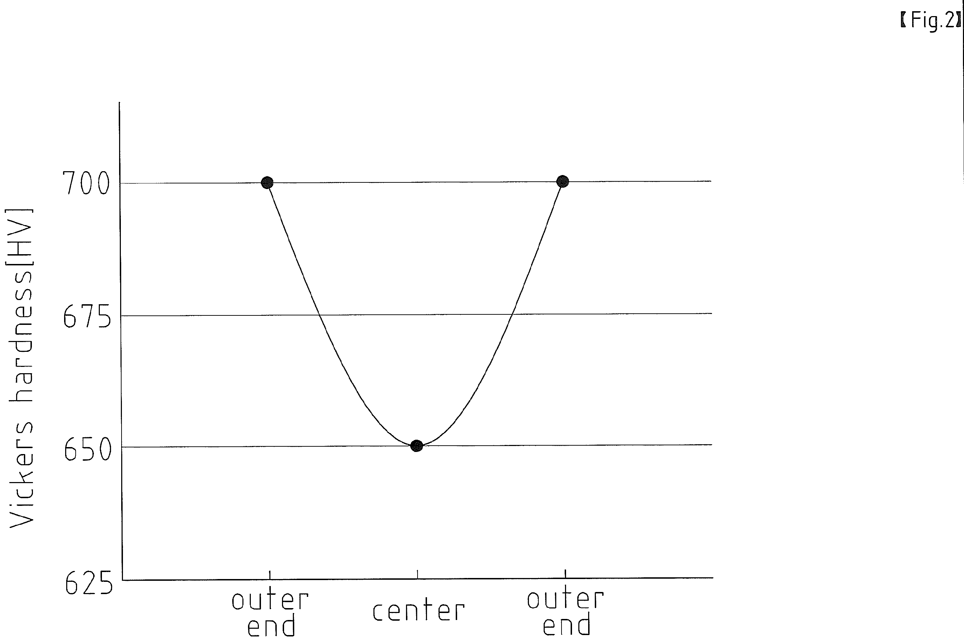

Further, Patent Document 2 describes a twisted wire rope having an improved life time, which can be prepared by forming an uneven surface on each surface of multiple metal element wires 16, and forming element twisted wires 17 by twisting the multiple metal element wires 16 each having the uneven surface, and twisting the element twisted wires 17 and then applying a resin coating 18 therearound, and filling spaces between the resin coating 18 and the element twisted wires 17 with grease (see Fig. 2 and others).

Further, Reference Document 3 describes an inner wire rope having an improved abrasion resistance and the like, which can be prepared by subjecting side strands to deforestation processing to make surface contacts between a core strand and the side strands, and sealing lubricating oil between the core strand and the side strands (see Fig. 1 and the like).

However, a step of grease filling is inevitably required when manufacturing the wire ropes described in Reference Documents 1 to 3 although grease to be contained in the wire ropes can improve their durability.

Further, the wire ropes described in Reference Documents 1 to 3 cannot be used in medical devices to be inserted into a patient's body because these wire ropes are filled with grease within the wire ropes. Therefore, a wire rope having an improved durability without containing grease has been desired to be developed for use in medical devices.

### CITATION LIST

### Patent Document

Patent Document 1: Japanese Patent Application Laid-Open No. 2004-327254
Patent Document 2: Japanese Patent Application Laid-Open No. H8-144182
Patent Document 3: Japanese Patent Application Laid-Open No. 2004-124342

### SUMMARY OF INVENTION

### TECHNICAL PROBLEM

The present invention is made in order to solve the above problem. An object of the present invention is to provide a wire rope having an improved durability without use of grease thereinside, in particular a wire rope with an improved durability which can be used in a medical device to be inserted into a patient's body.

### SOLUTION TO PROBLEM

In order to achieve the above object, a first aspect of the present invention is characterized by a wire rope formed by winding multiple metal element wires, in which at least one special metal element wire among the multiple metal element wires has a hardness of the outer periphery in a cross-section higher than that of the center in that cross-section.

Further, a second aspect of the present invention is characterized by the wire rope according to the first aspect of the invention, in which the at least one special metal element wire is arranged at the center.

Moreover, a third aspect of the present invention is characterized by the wire rope according to the second aspect of the invention, in which the multiple metal element wires consisting only of the at least one special metal element wire and multiple side metal element wires making contact with the special metal element wire.

Furthermore, a fourth aspect of the present invention is characterized by the wire rope according to the third aspect of the invention, in which the at least one special metal element wire has a circular cross-section, and the multiple side metal element wires each have an approximately trapezoidal cross-section.

Further, a fifth aspect of the present invention is characterized by a wire rope formed by twisting a plurality of wire ropes according to the fourth aspect of the invention.

Moreover, a sixth aspect of the present invention is characterized by the wire rope according to the first aspect of the invention, in which the at least one special metal element wire comprises multiple special metal element wires, and a twisted wire in which the multiple special metal element wires are twisted is arranged at the center.

### ADVANTAGEOUS EFFECT OF THE INVENTION

The first aspect of the present invention provides a wire rope formed by spirally winding multiple metal element wires, in which at least one special metal element wire among the multiple metal element wires has a hardness of the outer periphery in a cross-section higher than that of the center in that cross-section. This can provide an improved durability without use of grease thereinside, enabling its use in a medical device.

Further, the second aspect of the invention provides the wire rope according to the first aspect of the invention, in which the at least one special metal element wire is arranged at the center of the wire rope. This can further improve durability in addition to the effect of the first aspect of the invention.

Moreover, the third aspect of the invention provides the wire rope according to the second aspect of the invention, in which the multiple metal element wires consist only of the at least one special metal element wire, and multiple side metal element wires making contacts with the at least one special metal element wire. This can further improve durability in addition to the effect of the second aspect of the invention.

Furthermore, the fourth aspect of the invention provides the wire rope according to the third aspect of the invention, in which the at least one special metal element wire has a circular cross-section, and the multiple side metal element wires each have an approximately trapezoidal cross-section. This can further improve durability in addition to the effect of the third aspect of the invention.

Further, the fifth aspect of the invention provides a wire rope formed by twisting a plurality of wire ropes according to the fourth aspect. This can further improve durability.

Moreover, the sixth aspect of the invention provides the wire rope according to the first aspect of the invention, in which the at least one special metal element wire comprises multiple special metal element wires, and a twisted wire in which the multiple special metal element wires are twisted is arranged at the center. This can improve flexibility in addition to the effect of the first aspect of the invention.

### BRIEF DESCRIPTION OF DRAWINGS

Fig. 1 shows a cross-sectional view of a wire rope according to the first embodiment of the present invention.
Fig. 2 shows a first hardness distribution in a cross-section of a special metal element wire used for the wire rope according to the first embodiment.
Fig. 3 shows a second hardness distribution in a cross-section of a special metal element wire used for the wire rope according to the first embodiment.
Fig. 4 shows a side view of a wire rope according to the second embodiment of the present invention.
Fig. 5 shows a cross-sectional view at A-A in Fig. 4.
Fig. 6 shows a side view of a wire rope according to the third embodiment.
Fig. 7 shows a cross-sectional view at B-B in Fig. 6.
Fig. 8 shows a cross-sectional view of a wire rope according to the fourth embodiment.
Fig. 9 shows a cross-sectional view of a wire rope according to the fifth embodiment.

### DESCRIPTION OF EMBODIMENTS

Below, the aforementioned embodiments of the present invention will be described with reference to the drawings.

### First embodiment

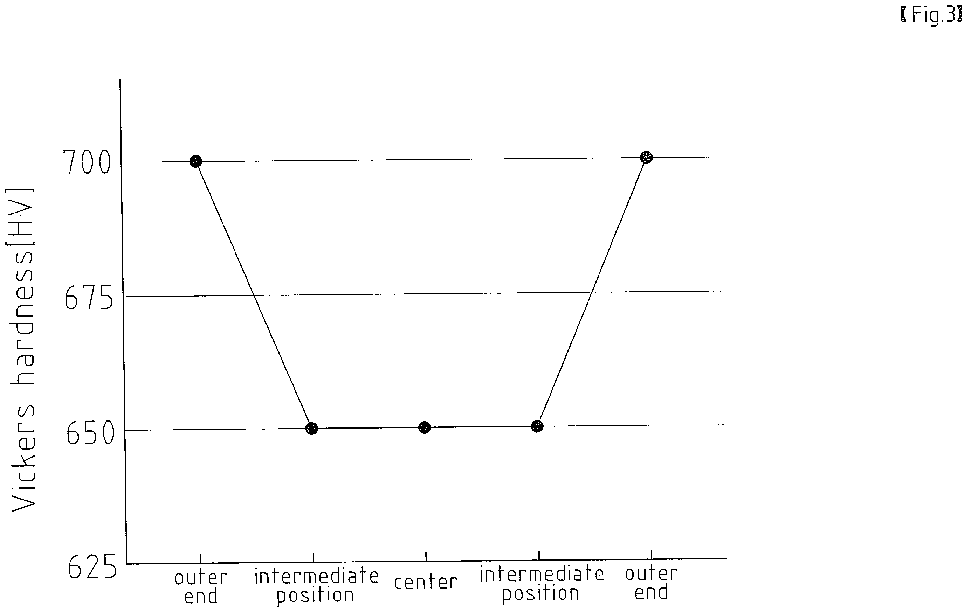

Fig. 1 shows a cross-sectional view of the wire rope according to the first embodiment of the present invention. Fig. 2 shows a first hardness distribution of a cross-section of a special metal element wire used for the wire rope according to first embodiment. Fig. 3 shows a second hardness distribution of a cross-section of a special metal element wire used for the wire rope according to first embodiment.

With reference to Fig. 1, a wire rope 1 comprises a core wire 3 (which corresponds to the "special metal element wire" according to the present invention) located at the center, and 6 side wires 5 (5a, 5b, 5c, 5d, 5e, and 5f) wound around the core wire 3.

The core wire 3 is a metal element wire having a circular cross-section extending from the front end to the base end through the center of the wire rope 1. There is no particular limitation for the material of the core wire 3, but stainless steel is used in the present embodiment.

Here, the core wire 3 has a hardness of the peripheral part in a cross-section higher than that of the center in the cross-section. That is, the core wire 3 is configured to have a structure where only the surface of the core wire 3 is hardened, but the inside of the core wire 3 is not hardened. This structure successfully serves not only to maintain the flexibility of the core wire 3 but also to improve the resistance to abrasion due to contacts with the side wires 5.

Note that conventionally known methods such as swaging and drawing dies can be used in order to obtain a hardness of the peripheral part in a cross-section of the metal element wire higher than that of the center in the cross-section.

Further, the structure in which only the surface of the core wire 3 is hardened, but the inside of the core wire 3 is not hardened may be, for example, a structure in which the hardness of the core wire 3 increases in the second-order fashion toward the outer periphery from the center in a cross-section of the wire rope 1 as shown in Fig. 2, or may be a structure in which the hardness increases in the linear fashion, or may be a structure in which the hardness of the core wire 3 shows a flat region in the vicinity of the center of the wire rope 1, and increases toward the outer periphery from the flat region as shown in Fig. 3.

Note that the hardness described in Figs. 2 and 3 is expressed in the Vickers hardness as measured with a Vickers hardness meter, and has a unit of "HV."

The hardness of the core wire 3 in Fig. 2 is about 650 HV at the center in a cross-section of the wire rope 1 while it is about 700 HV at the outer periphery, showing a difference of 50 HV. The hardness of the core wire 3 in Fig. 3 is constant at about 650 HV in the vicinity of the center of the wire rope 1 while it is about 700 HV at the outer periphery, showing a difference of 50 HV.

Note that the experiments performed by the present applicant demonstrated that the flexibility and abrasion resistance was improved even when the hardness at the center in a cross-section was decreased to 550 HV, and the hardness at the outer periphery in the cross-section was decreased to 580 HV.

In contrast, the flexibility of the wire rope 1 was impaired and the durability decreased when the hardness of the entire region in a cross-section of the wire rope 1 was, for example, 700 HV.

The side wires 5 (5a, 5b, 5c, 5d, 5e, and 5f), which are metal element wires each having a circular cross-section, are spirally wound around the core wire 3 in the longitudinal direction. There is no particular limitation for the material of the side wires 5 (5a, 5b, 5c, 5d, 5e, and 5f) as well, but stainless steel is used in the present embodiment. In addition to this, tungsten may also be used.

In the wire rope 1 according to the present embodiment, the core wire 3 having a hardness of the peripheral part in a cross-section higher than that of the center is arranged at the center of the wire rope 1 such that the multiple wires 5 (5a, 5b, 5c, 5d, 5e, and 5f) all make contacts with the core wire 3. This can improve the durability of the wire rope.

### Second embodiment

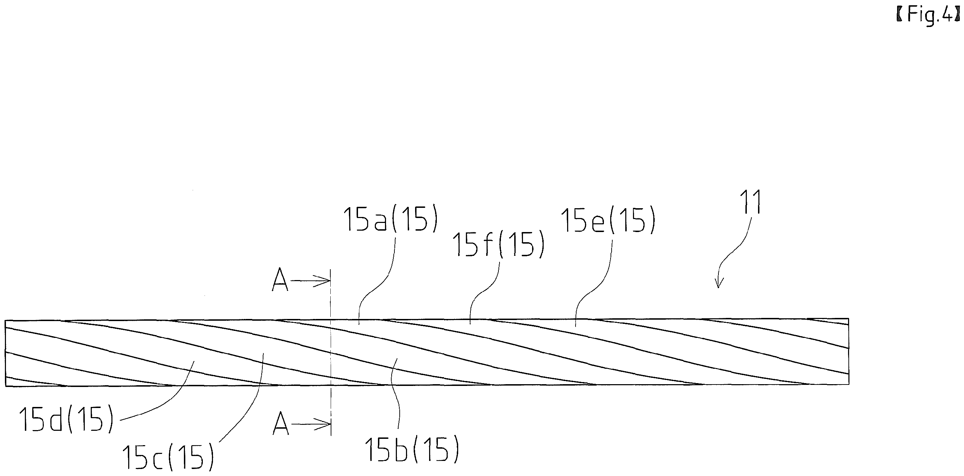

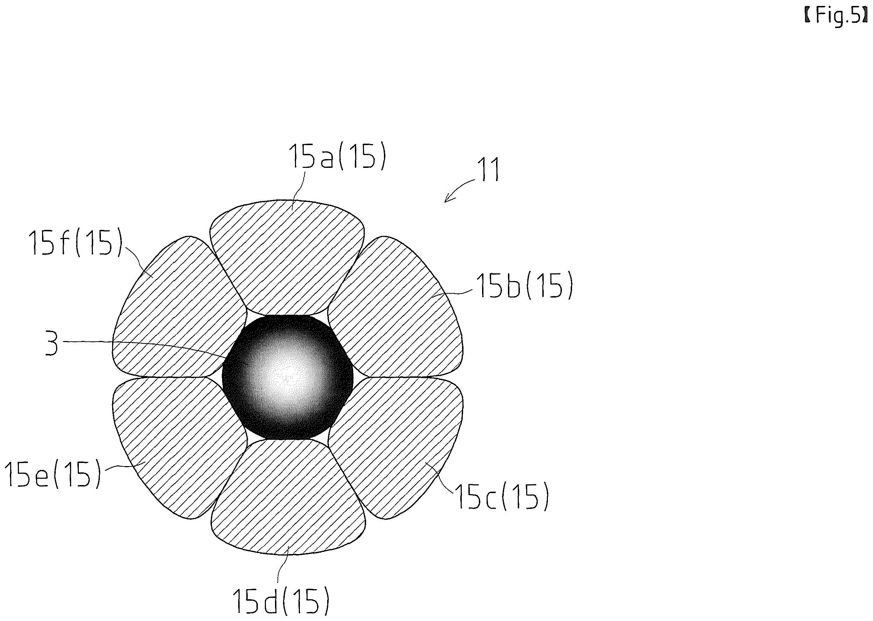

Below, a second embodiment of the present invention will be described. Descriptions will be omitted for the parts common with the first embodiment, to which the same reference numbers will be assigned in the figures. Fig. 4 shows a side view of a wire rope according to the second embodiment, and Fig. 5 shows a cross-sectional view at A-A in Fig. 4.

With reference to Figs. 4 and 5, a wire rope 11 comprises a core wire 3 located at the center, and 6 side wires 15 (15a, 15b, 15c, 15d, 15e, and 15f) wound around the core wire 3.

The side wires 15 (15a, 15b, 15c, 15d, 15e, and 15f), which are metal element wires each deforestation-processed into an approximately trapezoidal shape, are spirally wound around the core wire 3 in the longitudinal direction. There is no particular limitation for the material of the side wires 15 (15a, 15b, 15c, 15d, 15e, and 15f), but stainless steel is used in the present embodiment. In addition to this, tungsten may also be used.

In the wire rope 11 according to the present embodiment, the core wire 3 having a hardness of the peripheral part in a cross-section higher than that of the center is arranged at the center of the wire rope 1 such that the 6 side wires 5 (5a, 5b, 5c, 5d, 5e, and 5f) configured to be approximately trapezoidal and each having an approximately trapezoidal cross-section all make surface contacts with the core wire 3, and the wire rope 11 is configured to have an approximately circular cross-sectional outer periphery. This can improve not only the torque transmissibility of the wire rope 11 (the torque transmissibility to one end of a wire rope when the other end of the wire rope is rotated) but also the durability of the wire rope.

### Third embodiment

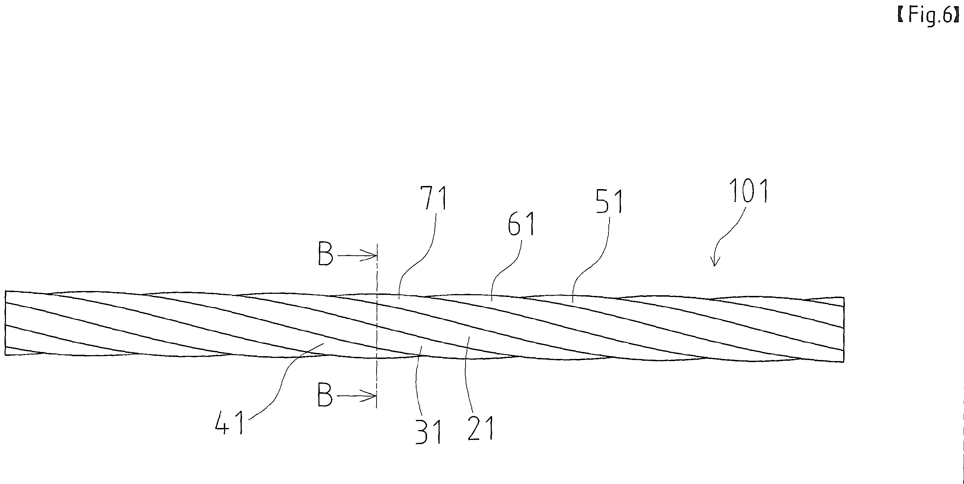

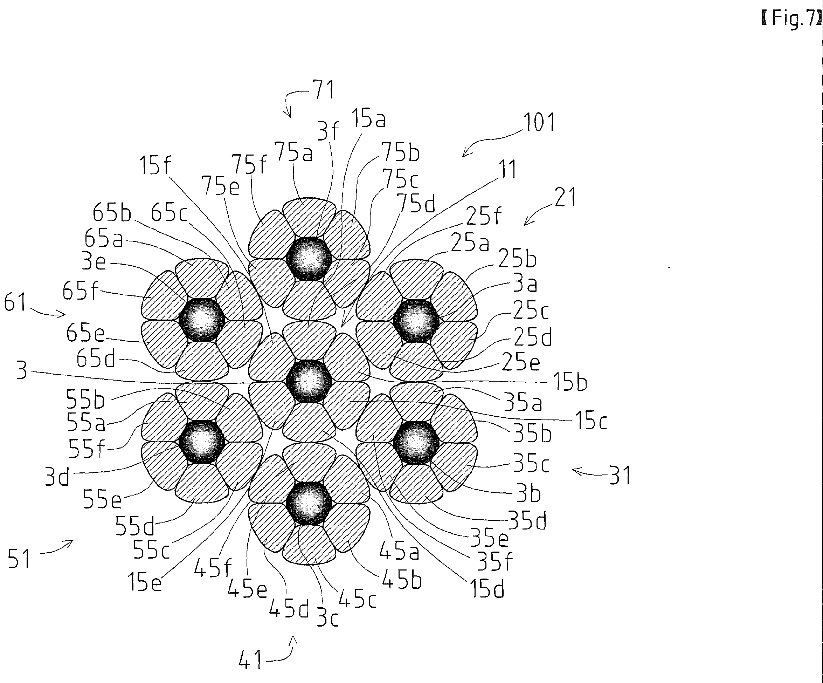

Below, a third embodiment of the present invention will be described. Descriptions will be omitted for the parts common with the first embodiment, to which the same reference numbers will be assigned in the figures. Fig. 6 shows a side view of a wire rope according to the third embodiment, and Fig. 7 shows a cross-sectional view at B-B in Fig. 6.

With reference to Figs. 6 and 7, a wire rope 101 comprises the core wire rope 11 according to the second embodiment located at the center, and 6 side wire ropes 21, 31, 41, 51, 61, and 71 wound around the core wire rope 11.

The side wire ropes 21, 31, 41, 51, 61, and 71 each have a similar structure to that of the core wire rope 11, and are spirally wound around the core wire rope 11 in the longitudinal direction.

That is, the side wire rope 21 comprises a core wire 3a located at the center (which corresponds to the "special metal element wire" according to the present invention) and 6 side wires 25 (25a, 25b, 25c, 25d, 25e, and 25f) wound around the core wire 3a, and the side wire rope 31 comprises a core wire 3b (which corresponds to the "special metal element wire" according to the present invention) located at the center and 6 side wires 35 (35a, 35b, 35c, 35d, 35e, and 35f) wound around the core wire 3b, and the side wire rope 41 comprises a core wire 3c (which corresponds to the "special metal element wire" according to the present invention) located at the center and 6 side wires 45 (45a, 45b, 45c, 45d, 45e, and 45f) wound around the core wire 3c.

Further, the side wire rope 51 comprises a core wire 3d (which corresponds to the "special metal element wire" according to the present invention) located at the center and 6 side wires 55 (55a, 55b, 55c, 55d, 55e, and 55f) wound around the core wire 3d, and the side wire rope 61 comprises a core wire 3e (which corresponds to the "special metal element wire" according to the present invention) located at the center and 6 side wires 65 (65a, 65b, 65c, 65d, 65e, and 65f) wound around the core wire 3e, and the side wire rope 71 comprises a core wire of 3f (which corresponds to the "special metal element wire" according to the present invention) located at the center and 6 side wires 75 (75a, 75b, 75c, 75d, 75e, and 75f) wound around the core wire 3f.

The wire rope 101 according to the present embodiment is formed by twisting a plurality of wire ropes in each of which a core wire having a hardness of the peripheral part in a cross-section higher than that of the center is arranged at the center such that 6 side wires each having an approximately trapezoidal cross-section all make contacts with the core wire, each wire rope being configured to have an approximately circular cross-sectional outer periphery. This can further improve not only the torque transmissibility of the wire rope 101 (the torque transmissibility to one end of a wire rope when the other end of the wire rope is rotated) but also the durability of the wire rope.

### Forth embodiment

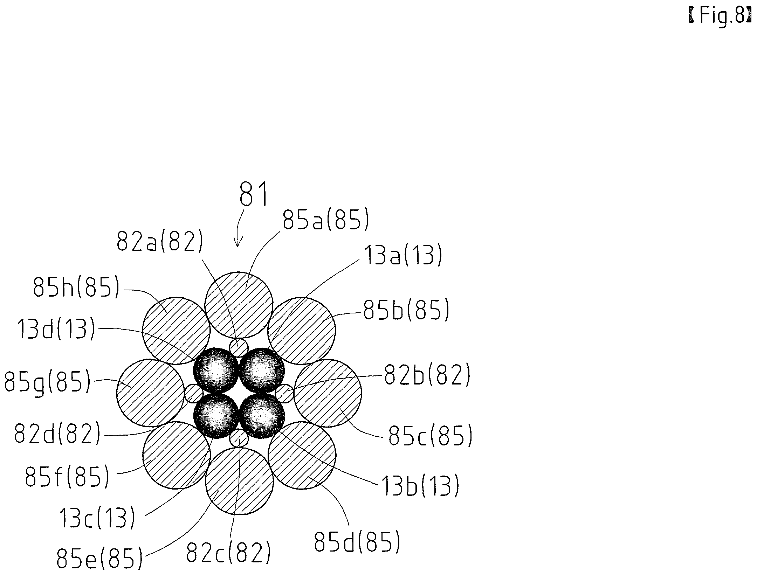

Below, a fourth embodiment will be described. Fig. 8 shows a cross-sectional view of a wire rope according to the fourth embodiment.

With reference to Fig. 8, a wire rope 81 comprises a core twisted wire 13 located at the center, 4 side wires 82 arranged at the outside of the core twisted wire 13, and 8 side wires 85 wound around the core twisted wire 13 and the side wires 82.

The core twisted wire 13 comprises 4 metal element wires (13a, 13b, 13c, and 13d (each corresponds to the "special metal element wire" according to the present invention)), and each metal element wire has a circular cross-section. There is no particular limitation for the material of the metal element wires (13a, 13b, 13c, and 13d), but stainless steel is used in the present embodiment.

Here, the metal element wires (13a, 13b, 13c, and 13d), which constitutes the core twisted wire 13, each have a hardness of the peripheral part in a cross-section higher than that of the central part in the cross-section. That is, each metal element wire (13a, 13b, 13c, and 13d) has a structure in which only the surface of the metal element wire is hardened, but the inside of the metal element wire is not hardened. Further, the core twisted wire 13 is formed by twisting four of this metal element wire. This can improve the flexibility of the core twisted wire 13, and can improve the durability.

Moreover, 4 side wires 82 (82a, 82b, 82c, and 82d), each of which has a circular cross-section, and comprises metal element wires each having a diameter smaller than that of each metal element wire (13a, 13b, 13c, and 13d) of the core twisted wire 13, is arranged at the outside of the core twisted wire 13. Note that there is no particular limitation for the material of the side wires 82 (82a, 82b, 82c, and 82d), but stainless steel is used in the present embodiment.

Further, 8 side wires 85 (85a, 85b, 85c, 85d, 85e, 85f, 85g, and 85h), which are metal element wires each having a circular cross-section, are arranged at the outside of the core twisted wire 13 and the side wires 82 (82a, 82b, 82c, and 82d). Note that there is no particular limitation for the material of the side wires 85 (85a, 85b, 85c, 85d, 85e, 85f, 85g, and 85h), but stainless steel is used in the present embodiment.

In the wire rope 81 according to the present embodiment, the core twisted wire 13 formed by twisting 4 metal element wires each having a hardness of the peripheral part in a cross-section higher than that of the central part is arranged at the center. This can further improve the flexibility of the wire rope 81, and can improve the durability.

### Fifth embodiment

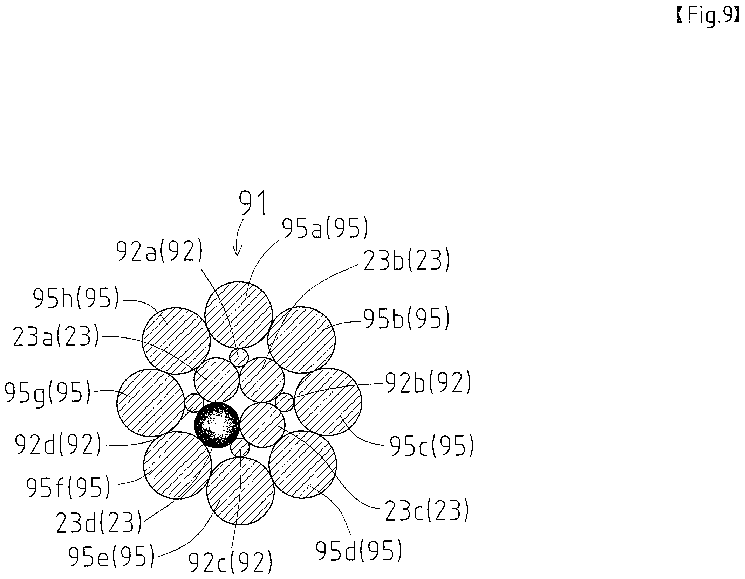

Below, a fifth embodiment will be described. Fig. 9 shows a cross-sectional view of a wire rope according to the fifth embodiment.

With reference to Fig. 9, a wire rope 91 comprises a core twisted wire 23 located at the center, 4 side wires 92 arranged at the outside of the core twisted wire 23, and 8 side wires 95 wound around the core twisted wire 23 and the side wires 92.

The core twisted wire 23 comprises 4 metal element wires (23a, 23b, 23c, and 23d (each corresponds to the "special metal element wire" according to the present invention)), and each of the metal element wire has a circular cross-section. There is no particular limitation for the material of the metal element wires (23a, 23b, 23c, and 23d), but stainless steel is used in the present embodiment.

Here, among the metal element wires (23a, 23b, 23c, and 23d) of the core twisted wire 23, the metal element wire 23d has a hardness of the peripheral part in a cross-section higher than that of the center in the cross-section. That is, the metal element wire 23d is configured to have a structure in which only the surface of the metal element wire is hardened, but the inside of the metal element wire is not hardened. On the other hand, the metal element wires 23a, 23b, and 23c each have an approximately constant hardness profile throughout a cross-section.

Further, the core twisted wire 23 is formed by twisting the 4 metal element wires. This can further improve the flexibility of the core twisted wire 23.

Moreover, 4 side wires 92 (92a, 92b, 92c, and 92d), each of which has a circular cross-section, and comprises metal element wires each having a diameter smaller than that of each metal element wires (23a, 23b, 23c, and 23d) of the core twisted wire 23, is arranged at the outside of the core twisted wire 23. Note that there is no particular limitation for the material of the side wires 92 (92a, 92b, 92c, and 92d), but stainless steel is used in the present embodiment.

Furthermore, 8 side wires 95 (95a, 95b, 95c, 95d, 95e, 95f, 95g, and 95h), which are metal element wires each having a circular cross-section, are arranged at the outside of the core twisted wire 23 and the side wire 92 (92a, 92b, 92c, and 92d). Note that there is no particular limitation for the material of the side wires 95 (95a, 95b, 95c, 95d, 95e, 95f, 95g, and 95h), but stainless steel is used in the present embodiment.

In the wire rope 91 according to the present embodiment, the core twisted wire 23 formed with the metal element wires each having a hardness of the peripheral part in a cross-section higher than that of the central part is arranged at the center of the wire rope 91. This can improve the flexibility and durability of the wire rope 91.

Although the wire ropes according to the various embodiments of the present invention are described above, the present invention shall not be limited to these embodiments. The present invention can be practiced with various modifications made without departing from the scope of the present invention.

For example, in the first embodiment to the third embodiment, the side wires 5, 15, 25, 35, 45, 55, 65, and 75 are each formed with 6 metal element wires. The number of metal element wires is, however, not limited to 6, and 3 or more may be sufficient.

Moreover, in the fourth and fifth embodiments, the core twisted wires 13 and 23 are each formed by twisting 4 metal element wires. The number of metal element wires is, however, not limited to 4, and two or more may be sufficient.

Moreover, in the fourth and fifth embodiments, the side wires 85 and 95 each comprise 8 metal element wires. The number of metal element wire is, however, not limited to 8, and any number may be used as long as the core twisted wire 13 or 23 is covered.

Moreover, in the fourth and fifth embodiments, the side wires 82 and 92 are provided, but the side wires 82 and 92 may not be present.

### DESCRIPTION OF SYMBOLS

- 1, 11, 21, 31, 41, 51, 61, 71, 81, 91, 101: Wire rope
- 3 Core: wire
- 13, 23: Twisted wire
- 5, 15, 25, 35, 45, 55, 65, 75, 82, 85, 92, 95: Side wire

## Claims

1. A wire rope formed by winding multiple metal element wires,
wherein at least one special metal element wire among the multiple metal element wires has a hardness of the outer periphery in a cross-section higher than that of the center in the cross-section.

2. The wire rope according to claim 1, wherein the at least one special metal element wire is arranged at the center.

3. The wire rope according to claim 2, wherein the multiple metal element wires consist only of the at least one special metal element wire and multiple side metal element wires making contacts with the at least one special metal element wire.

4. The wire rope according to claim 3, wherein the at least one special metal element wire has a circular cross-section, and the multiple side metal element wires each have an approximately trapezoidal cross-section.

5. A wire rope formed by twisting a plurality of wire ropes according to claim 4.

6. The wire rope according to claim 1, wherein the at least one special metal element wire comprises multiple special metal element wires, and a twisted wire in which the multiple special metal element wires are twisted is arranged at the center.
